(19) Europäisches Patentamt
European Patent Office
Office européen des brevets

(11) Numéro de publication: **0 042 794**
**B1**

(12) **FASCICULE DE BREVET EUROPEEN**

(45) Date de publication du fascicule du brevet: **14.12.83**

(21) Numéro de dépôt: **81400980.9**

(22) Date de dépôt: **18.06.81**

(51) Int. Cl.³: **C 07 C 69/73,**
C 07 C 67/333,
C 07 C 59/74,
C 07 C 51/373

(54) **4-méthyl 3-formyl penten-1-oates d'alcoyle, leur préparation, leur application à la préparation de l'acide 4-méthyl 3-formyl pen-3-èn-1-oique et les nouveaux intermédiaires obtenus.**

(30) Priorité: **20.06.80 FR 8013733**

(43) Date de publication de la demande:
**30.12.81 Bulletin 81/52**

(45) Mention de la délivrance du brevet:
**14.12.83 Bulletin 83/50**

(84) Etats contractants désignés:
**AT BE CH DE GB IT LI LU NL SE**

(56) Documents cités:
**EP - A - 0 023 849**

(73) Titulaire: **ROUSSEL-UCLAF**
**35, boulevard des Invalides**
**F-75007 Paris (FR)**

(72) Inventeur: **Martel, Jacques**
**15, rue Douvillez**
**F-93140-Bondy (FR)**
Inventeur: **Tessier, Jean**
**30, rue Jean Moulin**
**F-94300-Vincennes (FR)**
Inventeur: **Demoute, Jean-Pierre**
**249bis, rue de Rosny**
**F-93100-Montreuil sous Bois (FR)**

(74) Mandataire: **Burlot, Pierre et al,**
**ROUSSEL-UCLAF Boîte Postale No. 9 111, route de Noisy**
**F-93230 Romainville (FR)**

**EP 0 042 794 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

4-méthyl 3-formyl penten-1-oates d'alcoyle, leur préparation, leur application à la préparation de l'acide 4-méthyl 3-formyl pent-3-èn-1-oique et les nouveaux intermédiaires obtenus

L'invention concerne des 4-méthy 3-formyl penten-1-oates d'alcoyle, leur préparation, leur application à la préparation de l'acide 4-méthyl 3-formyl pent-3-èn-1-oique et les nouveaux intermédiaires obtenus.

L'invention a pour objet les composés de formule (I)

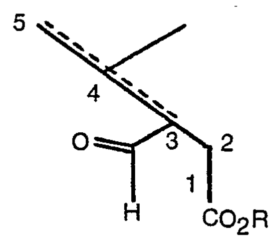

dans laquelle R représente un radical alcoyle comportant de 1 à 5 atomes de carbone et le trait pointillé représente une double liaison en position 3,4 ou 4,5.

Dans la formule générale (I) R peut représenter un radical méthyle, éthyle, propyle linéaire ou ramifié, butyle linéaire ou ramifié ou pentyle linéaire ou ramifié.

L'invention a notamment pour objet, parmi les composés de formule (I):
— le 4-méthyl 3-formyl pent-3-énoate de terbutyle,
— le 4-méthyl 3-formyl pent-4-énoate de terbutyle.

L'invention a également pour objet un procédé de préparation des composés de formule (I), caractérisé en ce que l'on fait réagir un dérivé azoté insaturé, substitué sur l'azote, de formule (II)

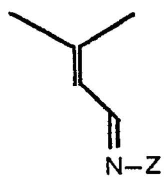

(II)

formule dans laquelle Z représente un radical alcoyle comportant de 1 à 6 atomes de carbone, un cycle carboné, un radical aryle monocyclique ou un radical

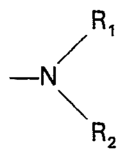

dans lequel $R_1$ et $R_2$ représentent un radical alcoyle comportant de 1 à 6 atomes de carbone, au sein d'un solvant et en présence d'une base forte ou d'un mélange de bases fortes, avec un halogéno-acétate d'alcoyle de formule (III)

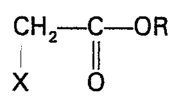

(III)

dans laquelle X représente un atome d'halogène et R représente un radical alcoyle comportant de 1 à 5 atomes de carbone, pour obtenir un composé de formule (IV)

(IV)

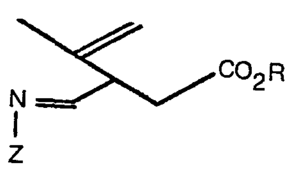

dans laquelle R et Z conservent les significations précitées, soumet ce composé (IV)
— soit à un double échange avec du formaldéhyde,
— soit à une hydrolyse en milieu acide,
pour obtenir un composé de formule ($I_1$)

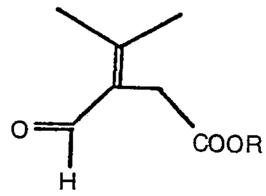

($I_1$)

ou un composé de formule ($I_2$)

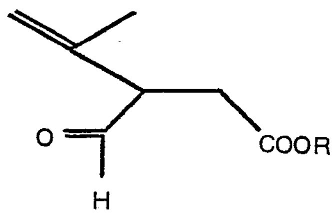

($I_2$)

ou un mélange de ces deux composés, composés dans lesquels R conserve les significations précitées.

Dans des conditions préférentielles d'exécution du procédé ci-dessus, la base forte ou le mélange de bases fortes servant à transformer les composés de formule (II) en composés de formule (IV) est choisi dans le groupe constitué par les organométalliques tels que les alcoyls ou aryllithiens, les amidures alcalins, les alcoolates alcalins et les hydrures alcalins et le solvant au sein duquel est effectuée la transformation du composé de formule (II) en composé de formule (IV) est choisi dans le groupe constitué par les solvants polaires aprotiques, notamment l'hexaméthyl phosphorotriamide, le diméthyl formamide, le tétrahydrofuran et le diméthoxyéthane, seuls ou en mélange avec des hydrocarbures.

Egalement selon une mise en oeuvre préféré du procédé, l'halogénoacétate d'alcoyle de formule (III) est choisi dans le groupe constitué par les chloroacétate et bromoacétate de méthyle, d'éthyle, de propyle, de n-butyle, de pentyle et de terbutyle.

L'invention a également pour objet à titre de produits industriels nouveaux et notamment à titre de produits intermédiaires nécessaires à la préparation des composés de formule (I), les composés de formule (IV) telle que définie précédemment et notamment le 4-diméthyl hydrazono 3-(propèn-2-yl) buténoate de terbutyle.

L'invention a enfin pour objet l'application des composés de formule (I) à la préparation de l'acide 4-méthyl 3-formyl pent-3-èn-1-oïque, application caractérisé en ce que l'on soumet le composé ($I_1$) ou le composé ($I_2$), ou leur mélange, à une acidolyse pour obtenir l'acide penténoïque désiré de formule

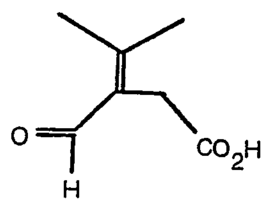

Dans des conditions préférentielles d'exécution du procédé de l'application ci-dessus l'on soumet le composé ($I_1$), le composé ($I_2$), ou leur mélange, à l'action d'un agent acide ou basique permettant l'hydrolyse de la fonction ester. Dans le cas de l'ester terbutylique, la libération de la fonction acide peut notamment être effectuée par action d'un acide fort en mlilieu anhydre. L'acide fort utilisé est de préférence l'acide trifluoroacétique.

L'acide 3-formyl 4-méthyl pent-3-èn-1-oïque est particulièrement utile parce qu'il permet de préparer les composés de formule ($\alpha$):

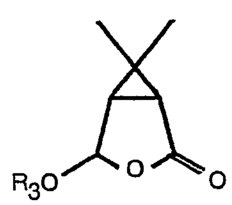

($\alpha$)

formule dans laquelle $R_3$ représente un atome d'hydrogène ou le reste d'un alcool $R_3OH$, par exemple selon le procédé décrit dans la demande de brevet européen publiée sous le N° 0023849 le 11 février 1981.

Les composés (α) servent, notamment à préparer les acides d'esters insecticides très actifs.

La partie expérimentale suivante illustre l'invention sans toutefois la limiter.

Exemple

4-méthyl 3-formyl pent-3-èn-1-oate de terbutyle et 4-méthyl 3-formyl pent-4-èn-1-oate de terbutyle.

*Stade A: 4-diméthyl hydrazono 3-(propèn-2-yl) buténoate de terbutyle.*

Dans un mélange de 25,8 m moles de diisopropylamine et de 5 volumes de tétrahydrofuran, on introduit à —20°/—30°C, 21,5 m moles de butyllithium en solution cyclohexanique, agite pendant 20 minutes à 20°/25°C, refroidit à +5°C, introduit goutte à goutte 17,9 m moles de 3-méthyl but-2-ène al -diméthylhydrazone en solution dans 2 volumes de tétrahydrofuran, agite pendant 20 minutes à + 20°C, refroidit à —15°C, introduit lentement une solution de 23,3 m moles de bromoacétate de terbutyle dans 2 volumes de tétrahydrofuran, laisse en contact une heure à cette température, verse le mélange réactionnel sur une solution glacée de dihydrogèno phosphate monosodique, extrait au benzène, concentre la liqueur benzénique à sec par distillation sous pression réduite, chromatographie le résidu sur gel de silice en éluant par un mélange de benzène et d'acétate d'éthyle (9/1) et obtient 2,4 g de 4-diméthyl hydrazono-3-(propen-2-yl) buténoate de terbutyle.

*Spectre IR* (chloroforme)

Absorption à 1645 et 900 $cm^{-1}$ attribués à $H_2C=C<$

Absorption à 1720 $cm^{-1}$ attribuée à $O=C$

*Spectre de RMN* (deutéro chloroforme)

— pic à 1,43 ppm attribué aux hydrogènes des méthyles du terbutyle,

— pic à 1,73 ppm attribué aux hydrogènes du méthyle en α du méthylène,

— pics à 2,43 — 2,61 ppm attribués aux hydrogènes en α du carbonyle,

— pic à 2,73 ppm attribué aux hydrogènes des méthyles de

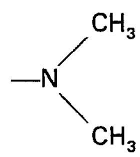

— pic à 3,33 ppm attribué à l'hydrogène en β du carboxyle,

— pic à 4,8 ppm attribué aux hydrogènes du méthylène

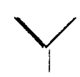

— pics à 6,47 — 6,55 ppm attribués à l'hydrogène

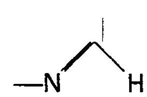

*Stade B: 4-méthyl 3-formyl pent-3-énoate de terbutyle et 4-méthyl 3-formyl pent-4-énoate de terbutyle.*

Dans un mélange de 1,5 cm3 d'acétone, 1 cm3 d'une solution de formaldéhyde à 40% dans l'eau, 0,1 cm3 de solution aqueuse d'acide chlorhydrique à 22°bé, on introduit 50 mg de 4-diméthyl hydrazono 3-(propen-2-yl) buténoate de terbutyle, agite à 20°C pendant 7 heures, extrait au benzène, concentre à sec par distillation sous pression réduite, purifie le produit brut par chromatographie sur gel de silice en éluant avec un mélange de benzène et d'acétate d'éthyle (9/1) et récupère 2 fractions:

Fraction A: 20 mg, de forme non conjuguée (composé $I_1$)

Fraction B: 12 mg, de forme conjuguée (composé $I_2$)

*Fraction A:* (composé $I_1$)

*Spectre IR* (chloroforme)

Absorption à 2715 $cm^{-1}$ attribuée à —CH de l'aldéhyde,

Absorption à 1720 $cm^{-1}$ attribuée à

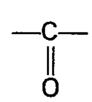

ester et aldéhyde,

Absorption à 1368 $cm^{-1}$ attribuée au t-butyle
Absorption à 905 $cm^{-1}$ attribuée à

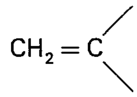

*Spectre de RMN* (deutérochloroforme)
— Pic à 1,45 ppm attribué aux hydrogènes des méthyles du terbutyle,
— pic à 1,75 ppm attribué aux hydrogènes du méthyle en position 5
— pic de 1,92 à 2,2 ppm attribué aux hydrogènes du méthylène situé en $\alpha$ du carboxyle,
— pic à 3,5 ppm attribué à l'hydrogène en position 3,
— pics à 4,3—8,4 ppm attribués aux hydrogènes terminaux de la double liaison.

*Fraction B:* (Composé $I_2$)
*Spectre IR* (chloroforme)
Absorption à 1721 $cm^{-1}$ attribuée à

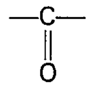

Absorption à 1662 $cm^{-1}$ attribuée à la fonction aldéhyde conjuguée,
Absorption à 1631 $cm^{-1}$ attribuée à C = C conjuguée,
Absorption à 1390 $cm^{-1}$ attribuée au gem diméthyle,
Absorption à 1370 $cm^{-1}$ attribuée au t-butyle.

*Spectre de RMN* (deutéro chloroforme)
— pic à 1,43 ppm attribué aux hydrogènes des méthyles du terbutyle,
— pic à 1,97 ppm attribué aux hydrogènes du méthyle en 5,
— pic à 2,25 ppm attribué aux hydrogènes du méthyle terminal,
— pic à 3,3 ppm attribué aux hydrogènes du méthylène en $\alpha$ du carboxyle,
— pic à 10,1 ppm attribué à l'hydrogène de l'aldéhyde.

Application: Acide 4-méthyl 3-formyl pent-3-èn-1-oïque.

Dans un mélange de 20 cm3 de chlorure de méthylène et 15,5 cm3 d'acide trifluoroacétique, refroidi à 0/5°C, on introduit 0,956 g de 4-méthyl 3-formyl pent-3-énoate de terbutyle, en solution dans 8,7 cm3 de chlorure de méthylène, agite pendant 3 heures à + 5°C, ajoute du cyclohexane dans le milieu réactionnel et évapore sous pression réduite. On purifie le produit brut cristallisé par empâtage dans l'ether isopropylique, obtient 0,444 g d'acide 4-méthyl 3-formyl pent-3-èn-1-oïque, F = 102°C. Par chromatographie des liqueurs mères sur gel de silice en éluant par une mélange de benzène, d'acétone et de chloroforme (1/1/1) on obtient 40 mg d'acide 4-méthyl 3-formyl pent-3-èn-1-oïque F = 102°C.

*Spectre de RMN*
— pic à 2,0 ppm attribué aux hydrogènes en 5,
— pic à 2,26 ppm attribué aux hydrogènes du méthyle en 4,
— pic à 3,38 ppm attribué aux hydrogènes en 2,
— pic à 10,1 ppm attribué à l'hydrogène du formyle.

De manière analogue au départ du 4-méthyl 3-formyl pent-4-énoate de terbutyle (forme B du stade B de l'exemple) on obtient l'acide 4-méthyl 3-formyl pent-3-èn-1-oïque.

**Revendications pour les Etats contractants: BE CH DE GB IT LI LU NL SE**

1. Les composés de formule (I)

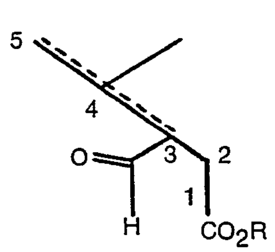

dans laquelle R représente un radical alcoyle comportant de 1 à 5 atomes carbone et le trait pointillé représente une double liaison en position 3,4 ou 4,5.

2. le 4-méthyl 3-formyl pent-3-énoate de terbutyle.

3. le 4-méthyl 3-formyl pent-4-énoate de terbutyle.

4. Procédé de préparation des composés de formule (I), telle que définie à la revendication 1, caractérisé en ce que l'on fait réagir un dérivé azoté insaturé, substitué sur l'azote, de formule (II)

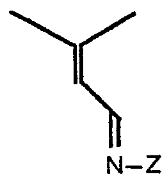

(II)

formule dans laquelle Z représente un radical alcoyle comportant de 1 à 6 atomes de carbone, un cycle carboné, un radical aryle monocyclique ou un radical

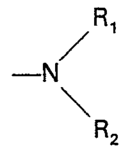

dans lequel $R_1$ et $R_2$ représentent un radical alcoyle comportant de 1 à 6 atomes de carbone, au sein d'un solvant et en présence d'une base forte ou d'un mélange de bases fortes, avec un halogénoacétate d'alcoyle de formule (III)

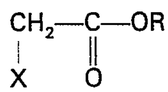

(III)

dans laquelle X représente un atome d'halogène et R est défini comme à la revendication 1, pour obtenir un composé de formule (IV)

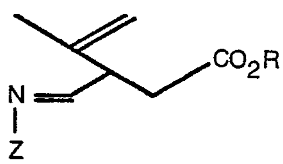

(IV)

dans laquelle R et Z conservent les significations précitées, soumet ce composé (IV)

— soit à un double échange avec du formaldéhyde,

— soit à une hydrolyse en milieu acide,

pour obtenir un composé de formule ($I_1$)

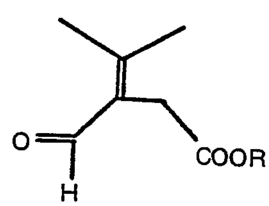

($I_1$)

ou un composé de formule ($I_2$)

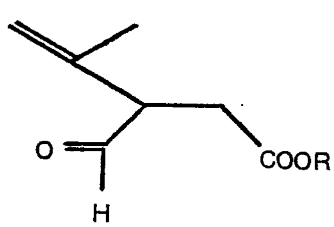

($I_2$)

ou un mélange de ces deux composés, composés dans lequels R conserve les significations précitées.

5. Procédé de préparation selon la revendication 4, caractérisé en ce que la base forte ou le mélange de bases fortes servant à transformer les composés de formule (II) en composés de formule (IV) sont choisis dans le groupe constitué par les organométalliques tels que les alcoyle -ou aryllithiens, les amidures alcalins, les alcoolates alcalins et les hydrures alcalins.

6. Procédé de préparation selon la revendication 4, caractérisé en ce que le solvant au sein duquel est effectuée la transformation du composé de formule (II) en composé de formule (IV) est choisi dans le groupe constitué par l'hexaméthyl phosphorotriamide, le diméthylformamide, le tétrahydrofurane et le diméthoxyéthane, seuls ou en mélange avec les hydrocarbures.

7. Procédé de préparation selon la revendication 4, caractérisé en ce que l'halogénoacétate d'alcoyle de formule (III) est choisi dans le groupe constitué par les chloroacétate et bromoacétate de méthyle, d'éthyle, de propyle, de n-butyle, de pentyle et de terbutyle.

8. Application des composés de formule (I) tels que définis à la revendication 1, à la préparation de l'acide 4-méthyl 3-formyl pent-3-èn-1-oïque, caractérisée en ce que l'on soumet le composé $(I_1)$ ou le composé $(I_2)$, ou leur mélange, à une acidolyse pour obtenir l'acide penténoïque désiré de formule

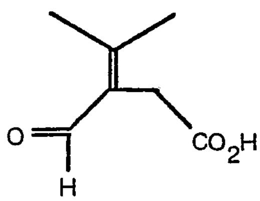

9. Application selon la revendication 8, caractérisé en ce que l'on soumet le composé $(I_1)$, le composé $(I_2)$ ou leur mélange, à l'action d'un agent acide ou basique permettant l'hydrolyse de la fonction ester, ou dans le cas de l'ester terbutylique à l'action d'un acide fort en milieu anhydre.

10. Application selon la revendication 8, caractérisé en ce que l'acide fort utilisé en milieu anhydre est l'acide trifluoroacétique.

11. A titre de produits industriels nouveaux nécessaires à la mise en oeuvre du procédé de la revendication 4, les composés de formule générale (IV)

12. A titre de produit industriel nouveau nécessaire à la mise en oeuvre du procédé de la revendication 4, le 4-diméthyl hydrazono 3-(propèn-2-yl) buténoate de terbutyle.

**Revendications pour l'Etat contractant: AT**

1. Procédé de préparation de l'acide 4-méthyl 3-formyl pent-3-èn-1-oïque, de formule

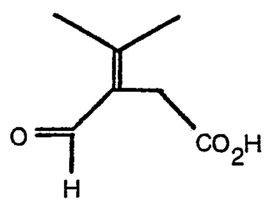

caractérisé en ce que l'on fait réagir un dérivé azoté insaturé, substitué sur l'azote, de formule (II)

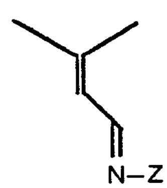

(II)

formule dans laquelle Z représente un radical alcoyle comportant de 1 à 6 atomes de carbone un cycle carboné, un radical aryle monocyclique ou un radical

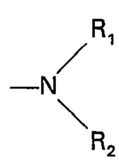

dans lequel $R_1$ et $R_2$ représentent un radical alcoyle comportant de 1 à 6 atomes de carbone, au sein d'un solvant et en présence d'une base forte ou d'un mélange de bases fortes, avec un halogéno-

acétate d'alcoyle de formule (III)

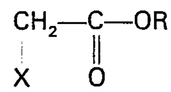

(III)

dans laquelle X représente un atome d'halogène et R représente un radical alcoyle comportant de 1 à 5 atomes de carbone,
pour obtenir un composé de formule (IV)

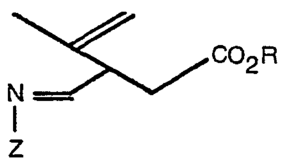

(IV)

dans laquelle R et Z conservent les significations précitées, soumet ce composé (IV)
— soit à un double échange avec du formaldéhyde,
— soit à une hydrolyse en milieu acide,
pour obtenir un composé de formule ($I_1$)

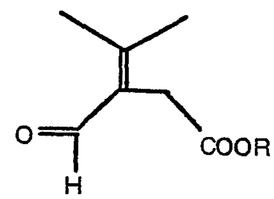

($I_1$)

ou un composé de formule ($I_2$)

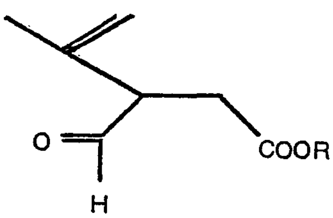

($I_2$)

ou un mélange de ces deux composés, composés dans lesquels R conserve les significations précitées, puis soumet le composé ($I_1$), le composé ($I_2$), ou leur mélange, à une acidolyse pour obtenir l'acide 4-méthyl 3-formyl pent-3-én-1-oïque désiré.

2. Procédé de préparation selon la revendication 1, caractérisé en ce que la base forte ou le mélange de bases fortes servant à transformer les composés de formule (II) en composés de formule (IV) sont choisis dans le groupe constitué par les organométalliques tels que les alcoyls -ou aryllithiens, les amidures alcalins, les alcoolates alcalins et les hydrures alcalins.

3. Procédé de préparation selon la revendication 1, caractérisé en ce que le solvant au sein duquel est effectué la transformation du composé de formule (II) en composé de formule (IV) est choisi dans le groupe constitué par l'hexaméthyl phosphorotriamide, le diméthyl formamide, le tétrahydrofurane et le diméthoxyéthane, seuls ou en mélange avec des hydrocarbures.

4. Procédé de préparation selon la revendication 1, caractérisé en ce que l'halogénoacétate d'alcoyle de formule (III) est choisi dans le groupe constitué par les chloroacétate et bromoacétate de méthyle, d'éthyle, de propyle, de n-butyle, de pentyle et de terbutyle.

5. Procédé de préparation selon la revendication 1, caractérisé en ce que l'on soumet le composé ($I_1$), le composé ($I_2$) ou leur mélange à l'action d'un agent acide ou basique permettant l'hydrolyse de la fonction ester, ou dans le cas de l'ester terbutylique, à l'action d'un acide fort en milieu anhydre.

6. Procédé de préparation selon la revendication 5, caractérisé en ce que l'acide fort utilisé en milieu anhydre est l'acide trifluoroacétique.

**Patentansprüche für die Vertragsstaaten: BE CH DE GB IT LI LU NL SE**

1. Verbindungen der Formel I

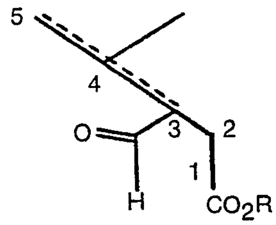

worin R einen Alkylrest mit 1 bis 5 Kohlenstoffatomen bedeutet und die gestrichelte Linie eine Doppelbindung in 3,4- oder 4,5-Stellung anzeigt.

2. 4-Methyl-3-formyl-pent-3-ensäure-tert.-butylester.

3. 4-Methyl-3-formyl-pent-4-ensäure-tert.-butylester.

4. Verfahren zur Herstellung von Verbindungen der Formel I gemäß Anspruch 1, dadurch gekennzeichnet, daß man ein ungesättigtes, an dem Stickstoff substituiertes Stickstoffderivat der Formel II

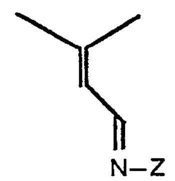

(II)

worin Z einen Alkylrest mit 1 bis 6 Kohlenstoffatomen, einen Kohlenstoffring, einen monocyclischen Arylrest oder einen Rest

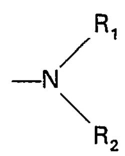

worin $R_1$ und $R_2$ einen Alkylrest mit 1 bis 6 Kohlenstoffatomen darstellen, bedeutet, in dem Medium eines Lösungsmittels und in Gegenwart einer starken Base oder eines Gemisches von starken Basen mit einem Halogenessigsäurealkylester der Formel III

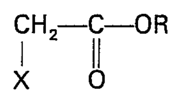

(III)

worin X ein Halogenatom bedeutet und R wie in Anspruch 1 definiert ist, umsetzt, um eine Verbindung der Formel IV

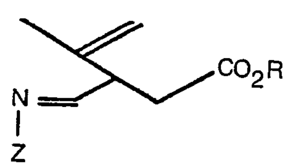

(IV)

worin R und Z die vorstehend angegebenen Bedeutungen besitzen, zu erhalten,
diese Verbindung IV
entweder einem zweifachen Austausch mit Formaldehyd oder einer Hydrolyse in saurem Milieu unterzieht, um eine Verbindung der Formel $I_1$

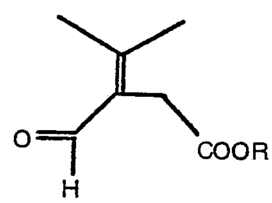

($I_1$)

oder eine Verbindung der Formel $I_2$

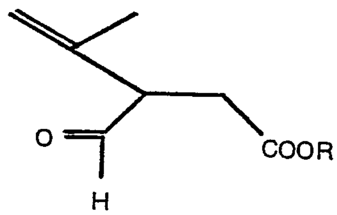

$(I_2)$

oder ein Gemisch dieser beiden Verbindungen zu erhalten, in denen R die vorstehend angegebenen Bedeutungen besitzt.

5. Verfahren gemäß Anspruch 4, dadurch gekennzeichnet, daß die starke Base oder das Gemisch der starken Basen, die der Überführung der Verbindungen der Formel II in Verbindungen mit der Formel IV dienen, unter den organometallischen Verbindungen wie den Alkyl- oder Aryllithiumverbindungen, den Alkaliamiden, den Alkalialkoholaten und den Alkalihydriden ausgewählt werden.

6. Verfahren gemäß Anspruch 4, dadurch gekennzeichnet, daß das Lösungsmittel, in dessen Medium die Überführung der Verbindung der Formel II in die Verbindung der Formel IV durchgeführt wird, unter Hexamethylphosphortrisamid, Dimethylformamid, Tetrahydrofuran und Dimethoxyäthan allein oder im Gemisch mit Kohlenwasserstoffen ausgewählt wird.

7. Verfahren gemäß Anspruch 4, dadurch gekennzeichnet, daß der Halogenessigsäurealkylester der Formel III unter Methyl-, Äthyl-, Propyl-, n-Butyl-, Pentyl- und tert.-Butylchloracetat und -bromacetat ausgewählt wird.

8. Verwendung der Verbindungen der Formel I gemäß Anspruch 1 zur Herstellung der 4-Methyl-3-formyl-pent-3-en-1-säure, dadurch gekennzeichnet, daß man die Verbindung $I_1$ oder die Verbindung $I_2$ oder ihr Gemisch einer Acidolyse unterzieht, um die gewünschte Pentensäure der Formel

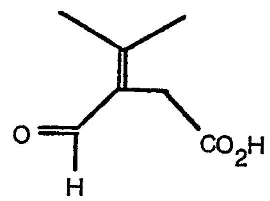

zu erhalten.

9. Verwendung gemäß Anspruch 8, dadurch gekennzeichnet, daß man die Verbindung $I_1$, die Verbindung $I_2$ oder ihr Gemisch der Einwirkung eines sauren oder basischen Mittels, die die Hydrolyse der Esterfunktion gestatten, oder im Fall des tert.-Butylesters der Einwirkung einer starken Säure in wasserfreiem Milieu unterzieht.

10. Verwendung gemäß Anspruch 8, dadurch gekennzeichnet, daß die in wasserfreiem Milieu verwendete starke Säure Trifluoressigsäure ist.

11. Als neue industrielle Produkte, die für die Durchführung des Verfahrens gemäß Anspruch 4 erforderlich sind, die Verbindungen der allgemeinen Formel IV.

12. Als neues industrielles Produkt, das für die Durchführung des Verfahrens gemäß Anspruch 4 erforderlich ist, der 4-Dimethylhydrazono-3-(propen-2-yl)-butensäure-tert.-butylester.

**Patentansprüche für den Vertragsstaat: AT**

1. Verfahren zur Herstellung der 4-Methyl-3-formyl-pent-3-en-1-säure der Formel

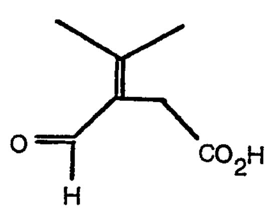

dadurch gekennzeichnet, daß man ein ungesättigtes, am Stickstoff substituiertes Stickstoffderivat der Formel II

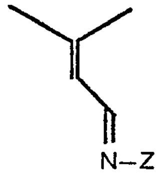

(II)

worin Z einen Alkylrest mit 1 bis 6 Kohlenstoffatomen, einen Kohlenstoffring, einen monocyclischen Arylrest oder einen Rest

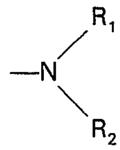

worin $R_1$ und $R_2$ einen Alkylrest mit 1 bis 6 Kohlenstoffatomen darstellen, bedeutet, in dem Medium eines Lösungsmittels und in Gegenwart einer starken Base oder eines Gemisches starker Basen mit einem Halogenessigsäurealkylester der Formel III

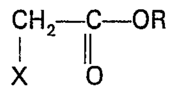

(III)

worin X ein Halogenatom bedeutet und R einen Alkylrest mit 1 bis 5 Kohlenstoffatomen darstellt, umsetzt, um eine Verbindung der Formel IV

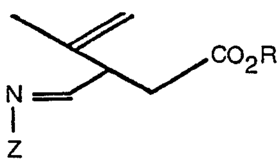

(IV)

worin R und Z die vorstehend angegebenen Bedeutungen besitzen zu erhalten,
diese Verbindung IV
entweder einem zweifachen Austausch mit Formaldehyd
oder einer Hydrolyse in saurem Milieu
unterzieht, um eine Verbindung der Formel $I_1$

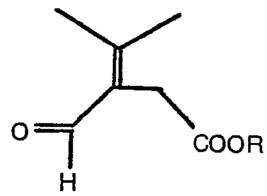

($I_1$)

oder eine Verbindung der Formel $I_2$

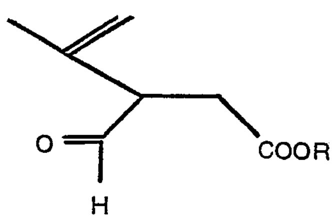

($I_2$)

oder ein Gemisch dieser beiden Verbindungen, worin R die vorstehend angegebenen Bedeutungen besitzt, zu erhalten, und danach die Verbindung $I_1$, die Verbindung $I_2$ oder ihr Gemisch einer Acidolyse unterzieht, um die gewünschte 4-Methyl-3-formyl-pent-3-en-1-säure zu erhalten.

2. Verfahren gemäß Anspruch 1, dadurch gekennzeichnet, daß die starke Base oder das Gemisch der starken Basen, die der Überführung der Verbindungen der Formel II in Verbindung mit der Formel IV dienen, unter den organometallischen Verbindungen wie den Alkyl- oder Aryllithiumverbindungen, den Alkaliamiden, den Alkalialkoholaten und den Alkalihydriden ausgewählt werden.

3. Verfahren gemäß Anspruch 1, dadurch gekennzeichnet, daß das Lösungsmittel, in dessen Medium die Überführung der Verbindung der Formel II in die Verbindung der Formel IV durchgeführt wird, unter Hexamethylphosphortrisamid, Dimethylformamid, Tetrahydrofuran und Dimethoxyäthan allein oder im Gemisch mit Kohlenwasserstoffen ausgewählt wird.

4. Verfahren gemäß Anspruch 1, dadurch gekennzeichnet, daß der Halogenessigsäurealkylester der Formel III unter Methyl-, Äthyl-, Propyl-, n-Butyl-, Pentyl- und tert.-Butylchloracetat und -bromacetat ausgewählt wird.

5. Verfahren gemäß Anspruch 1, dadurch gekennzeichnet, daß man die Verbindung $I_1$, die Verbindung $I_2$ oder ihr Gemisch der Einwirkung eines sauren oder basischen Mittels, das die Hydrolyse der Esterfunktion ermöglicht oder im Fall des tert.-Butylesters der Einwirkung einer starken Säure in wasserfreiem Milieu unterzieht.

6. Verfahren gemäß Anspruch 5, dadurch gekennzeichnet, daß die in wasserfreiem Milieu verwendete starke Säure Trifluoressigsäure ist.

**Claims for the Contracting States: BE CH DE GB IT LI LU NL SE**

1. Compounds with the formula (I):

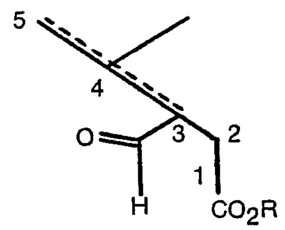

(I)

in which R represents an alkyl radical containing 1—5 carbon atoms, the broken line represents a double bond in position 3,4 or 4,5.

2. 4-methyl 3-formyl pent-3-enoate of tertbutyl.

3. 4-methyl 3-formyl pent-4-enoate of tertbutyl.

4. Process for the preparation of compounds with the formula (I) as defined in Claim 1, characterized in that an unsaturated nitrogenated derivative, substituted on the nitrogen, with formula (II)

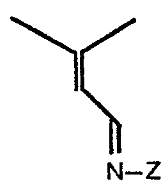

(II)

in which Z represents an alkyl radical containing 1—6 carbon atoms, a carbonated ring, a monocyclic aryl radical or a

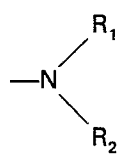

radical, in which $R_1$ and $R_2$ represent alkyl radicals containing 1—6 carbon atoms, in a solvent and in the presence of a strong base or a mixture of strong bases is made to react with an alkyl halogenoacetate with the formula (III)

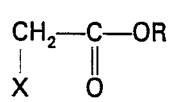

(III)

in which X represents a halogen atom and R is defined as in Claim 1 so as to obtain a compound with the formula (IV)

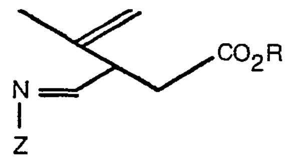

(IV)

in which R and Z retain their previous significance, this compound then being submitted

— either to a double exchange with formaldehyde
— or to hydrolysis in an acid medium

so as to obtain a compound with the formula ($I_1$)

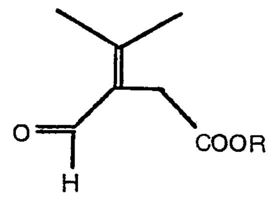

($I_1$)

or a compound with the formula ($I_2$)

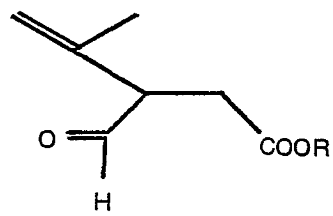

($I_2$)

or a mixture of these two compounds in which R retains its previous significance.

5. Process for the preparation according to Claim 4, characterized in that the strong base or mixture of strong bases being used to transform the compounds with the formula (II) into compounds with the formula (IV) are chosen from the group constituted by the organo-metallics such as alkyl- or aryl-lithiums, alkaline amides, alkaline alcoholates and alkaline hydrides.

6. Process for the preparation according to Claim 4, characterized in that the solvent in which the transformation of the compound with the formula (II) into the compound with the formula (IV) is carried out is chosen from the group constituted by hexamethyl phosphorotriamide, dimethylformamide, tetrahydrofuran and dimthoxyethane, alone or mixed with hydrocarbons.

7. Process for the preparation according to Claim 4, characterized in that the alkyl halogenoacetate with the formula (III) is chosen from the group constituted by the chloroacetates and bromoacetates of methyl, ethyl, propyl, n-butyl, pentyl and tert-butyl.

8. Application of the compounds with the formula (I) as defined in Claim 1 in the preparation of 4-methyl 3-formyl pent-3-1-oic acid, characterized in that the compound ($I_1$) or the compound ($I_2$) or their mixture is submitted to acidolysis so as to obtain the pentenoic acid desired with the formula

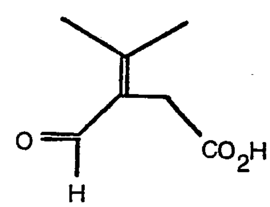

9. Application according to Claim 8, characterized in that the compound ($I_1$) or the compound ($I_2$) or their mixture is submitted to the action of an acid agent or base agent enabling hydrolysis of the ester function, or in the case of tert-butylic ester, to the action of a strong acid in anhydrous medium.

10. Application according to Claim 8, characterized in that the strong acid utilized in an anhydrous medium is trifluoroacetic acid.

11. As new industrial products necessary for the putting into operation of the process of Claim 4, the compounds with the general formula (IV).

12. As new industrial products for the putting into operation of the process in Claim 4, tert-butyl 4-dimethyl hydrazono 3-(propen-2-yl) butenoate.

**Claims for the Contracting State: AT**

1. Process for the preparation of 4-methyl 3-formyl pent-3-en-1-oic acid with the formula

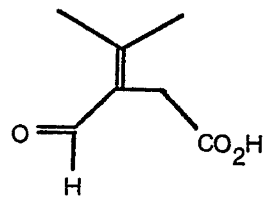

characterized in that an unsaturated nitrogenated derivative, substituted on the nitrogen, with formula (II)

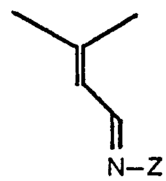

(II)

in which Z represents an alkyl radical containing 1—6 carbon atoms, a carbonated ring a monocyclic aryl radical or a

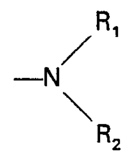

radical, in which $R_1$ and $R_2$ represent an alkyl radical containing 1—6 carbon atoms, in a solvent and in the presence of a strong base or a mixture of strong bases, is made to react with an alkyl halogenoacetate with the formula (III)

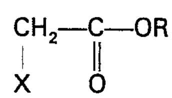

(III)

in which X represents a halogen atom and R represents an alkyl radical containing 1—5 carbon atoms, so as to obtain a compound with the formula (IV)

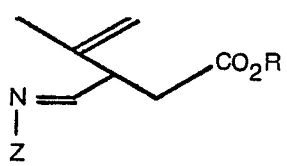

(IV)

in which R and Z retain their previous significance; this compound is then submitted

— either to a double exchange with formaldehyde
— or to hydrolysis in an acid medium

so as to obtain a compound with the formula ($I_1$)

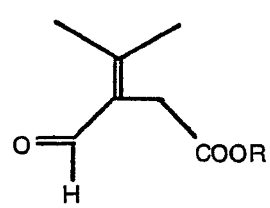

($I_1$)

or a compound with the formula ($I_2$)

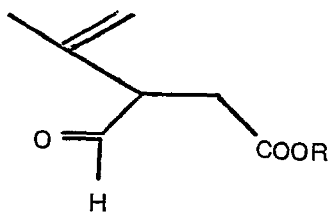

($I_2$)

or a mixture of these two compounds, in which R retains its previous significance, then compound ($I_1$) or compound ($I_2$) or their mixture, is submitted to acidolysis so as to obtain the 4-methyl 3-formyl pent-3-en-1-oic acid desired.

2. Process for the preparation according to Claim 1, characterized in that the strong base or mixture of strong bases being used to transform the compounds with the formula (II) into compounds with the formula (IV) are chosen from the group constituted by organo-metallics such as alkyl- or aryl-lithiums, alkaline amides, alkaline alcoholates and alkaline hydrides.

3. Process for the preparation according to Claim 1, characterized in that the solvent in which the transformation of the compound with the formula (II) into a compound with the formula (IV) is carried out is chosen from the group constituted by hexamethyl phosphorotriamide, dimethyl formamide, tetrahydrofuran and dimethoxyethane, alone or in a mixture with hydrocarbons.

4. Process for the preparation according to Claim 1, characterized in that the alkyl halogenoacetate with formula (III) is chosen from the group constituted by chloroacetates and bromoacetates of methyl, ethyl, propyl, n-butyl, pentyl and tert-butyl.

5. Process for the preparation according to Claim 1, characterized in that compound ($I_1$), compound ($I_2$) or their mixture is submitted to the action of an acid agent or base agent, enabling hydrolysis of the ester function, or in the case of the tert-butylic ester, to the action of a strong acid in an anhydrous medium.

6. Process for the preparation according to Claim 5, characterized in that the strong acid utilized in the anhydrous medium is trifluoracetic acid.